Europäisches Patentamt

**European Patent Office**

**Office européen des brevets**

(19)

(11) Veröffentlichungsnummer: **0 102 008**
**A2**

(12) # EUROPÄISCHE PATENTANMELDUNG

(21) Anmeldenummer: **83107954.6**

(22) Anmeldetag: **11.08.83**

(51) Int. Cl.³: **A 61 K 39/00**
**A 61 K 49/02, G 01 N 33/54**

(30) Priorität: **14.08.82 DE 3230299**

(43) Veröffentlichungstag der Anmeldung:
**07.03.84 Patentblatt 84/10**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lamerz, Rolf, Prof. Dr.**
**Wilhelm-Kuhnert-Strasse 13**
**D-8000 München 90(DE)**

(54) Carcinoembryonales Antigen, Verfahren zu seiner Isolierung und seine Verwendung.

(57) Carcinoembryonales Antigen (CEA) ist erhältlich aus CEA enthaltenden Perchlorsäureextrakten durch Abtrennung höhermolekularer Proteine durch Gelfiltration, Selektionierung der Proteine mit CEA-Antigenität aus dem Eluat durch Immunadsorption und Auftrennung durch Flüssigchromatographie an hochauflösenden Säulen. Es eignet sich zur Herstellung von Diagnostika.

EP 0 102 008 A2

HOECHST AKTIENGESELLSCHAFT    HOE 82/F 165    Dr.Q1/Q2008

Carcinoembryonales Antigen, Verfahren zu seiner
Isolierung und seine Verwendung

Beim carcinoembryonalen Antigen (CEA) handelt es sich um ein Glykoprotein mit elektrophoretischer $beta_1$-Mobilität und einem apparenten Molekulargewicht von ca. 200 000 Dalton, das in einer geringen Konzentration von einigen ng/ml im Serum und/oder Plasma von gesunden Probanden nachgewiesen werden kann, bei Patienten mit benignen Erkrankungen vor allem von Darm, Leber und Bauchspeicheldrüse transitorisch mäßig erhöhte Konzentrationen bis ca. 20-30 ng/ml oder konstant niedrig erhöhte Spiegel zeigt und typischerweise bei Patienten mit einer Vielzahl von malignen Erkrankungen (vorwiegend gastrointestinale Karzinome, Pankreaskarzinom, Lungenkarzinom, metastasierendes Mammakarzinom) im Tumor gebildet wird und mit deutlich bis stark erhöhten Konzentrationen (bis maximal über 10 000 ng/ml) im Blut oder anderen Körperflüssigkeiten einhergeht. Seine wesentliche klinische Bedeutung besteht in der Therapie- und Verlaufskontrolle (Operation, Hormo-, Radio- und Chemotherapie) von Tumorpatienten, da bei einem großen Teil von ihnen CEA-Spiegel-Veränderungen auftreten und oft der klinischen Entwicklung um Monate vorausgehen können (Übersicht in Klin. Wschr. 53, 193 - 203, 1975).

Eine Vielzahl von Methoden zur Extraktion, Reinigung und Isolierung von CEA sind in der wissenschaftlichen Literatur beschrieben (Übersicht s.o.). Am häufigsten wurde die Extraktion von Tumorgewebe durch Perchlorsäure angewandt. Die weitere Reinigung geschieht bei den meisten Verfahren durch Kombination eines Gelfitrations- mit einem Elektrophorese-Schritt, bei einigen Methoden auch durch zusätzliche Verwendung einer Immunadsorption.

Als Ausgangsmaterial eignet sich auch ein CEA aus einer entsprechenden Zellkultur.

Es wurde nun gefunden, daß ohne Elektrophorese-Schritt ein sehr reines CEA aus CEA enthaltenen Perchlorsäure-Extrakten erhalten wird, wenn

a) höhermolekulare Proteine durch Gelfiltration abgetrennt werden,

b) aus dem Eluat durch Immunadsorption Proteine mit CEA-Antigenität selektioniert werden, die

c) durch Flüssigchromatographie mit hochauflösenden (high performance) Säulen (im nachfolgenden bezeichnet als "Hochleistungschromatographie") aufgetrennt werden.

Das so erhältliche CEA ist elektrophoretisch heterogen, immunologisch jedoch einheitlich. Es eignet sich zur Herstellung radiojodmarkierter Derivate für diagnostische Zwecke.

Im folgenden werden bevorzugte Ausgestaltungen der Erfindung näher erläutert:

Die Abtrennung der höhermolekularen Proteine erfolgt durch Gelfiltration an hierfür geeigneten Säulen mittels quervernetzten Dextranen mit einem Proteinfraktionierbereich, der auch Proteine mit einem signifikant höheren Molgewicht als dem des CEA abtrennt. Das Eluat wird fraktioniert aufgefangen, der CEA-Gehalt mit üblichen analytischen Methoden ermittelt, die entsprechenden CEA-positiven Fraktionen vereinigt und konzentriert.

Je nach Gehalt des Extrakts an mit CEA kreuzreagierenden Komponenten (überwiegend NCA = nonspecific cross reacting antigen) erfolgt bereits auf dieser Stufe eine Vorreinigung unter Abtrennung des später eluierten (weil niedermolekularen) NCA.

Es ist möglich, jedoch nicht erforderlich, die Eluate durch einen weiteren Chromatographieschritt zu reinigen. Hierfür kann eine Säule mit engerem Trennbereich der Proteinfraktionen, beispielsweise mit einem Fraktionierbereich von $5 \cdot 10^3$ bis $5 \cdot 10^5$ D, eingesetzt werden. Hierbei kann oft eine bessere Trennung von CEA und NCA erreicht werden.

Die auf diese Weise von den höhermolekularen bzw. auch niedermolekularen Proteinen befreiten CEA-positiven Fraktionen werden im nächsten erfindungsgemäßen Schritt durch Immunadsorption aufgetrennt. Hierzu wird eine geeignete Adsorptionssäule mit vorgereinigten IgG-Globulinen eines polyklonalen Anti-CEA-Antiserums, das beispielsweise durch Immunisierung von Kaninchen oder Ziegen gewonnen wurde, oder eines monoklonalen Anti-CEA-Antiserums beladen. Auf diese Affinitäts-Chromatographiesäule werden die CEA-positiven Fraktionen aus dem vorhergehenden Schritt aufgegeben und zunächst die nicht-gebundenen Proteine eluiert. Anschließend werden mit einem Desorptionspuffer die Antikörper-gebundenen Proteine eluiert und die Eluate konzentriert.

Es ist möglich, eine zweite Affinitäts-Chromatographiesäule mit angekoppeltem normalem Perchlorsäure-Lungenextrakt mit einem polyklonalen Anti-CEA-Antiserum zu beschicken, wobei dann die Antikörper gegen NCA gebunden und aus dem Antiserum eliminiert werden.

Weiterhin ist es möglich, eine zusätzliche Affinitäts-Chromatographiesäule mit gekoppelten IgG-Antikörpern gegen humane Serumproteine wie Albumin, $alpha_1$-Antitrypsin und saures $alpha_1$-Glykoprotein herzustellen. Durch Chromatographie CEAhaltiger Präparationen an dieser Säule ist es möglich, Serumproteinverunreinigungen zu entfernen, welche sich bei der ersten Affinitätschromatographie unspezifisch adsorbiert hatten.

Die so erhaltenen Fraktionen enthalten neben intaktem CEA und NCA noch CEA-Bruchstücke, die ebenfalls mit den Anti-CEA-Antikörpern der Immunadsorptionssäule reagiert hatten. Zur weiteren Auftrennung schließt sich deshalb ein HPLC-Schritt an.

In dieser letzten Stufe werden an einer hochauflösenden HPLC-Säule die CEA-positiven Fraktionen aus der vorhergehenden Stufe aufgetrennt. Man erhält so die folgenden Fraktionen:

I. Molgewichtsbereich 250.000 bis 150.000: reines intaktes CEA,

II. Molgewichtsbereich 150.000 bis 80.000: immunoreaktive CEA-Bruchstücke (noch geeignet zur Immunisierung) und

III. Molgewichtsbereich 60.000 bis 40.000: intaktes NCA und niedermolekulare CEA-Bruchstücke.

Die Fraktion I kann nach Konzentration direkt für eine Radiojodmarkierung von CEA nach dem Chloramin T-Verfahren eingesetzt werden.

Die konzentrierten Fraktionen können mit Hilfe einer geeigneten Säule entsalzt und anschließend lyophylisiert werden.

In den folgenden Beispielen wird die Erfindung näher erläutert. Prozentangaben beziehen sich hierbei auf das Gewicht, sofern nichts anderes angegeben ist.

Der in den Beispielen eingesetzte CEA-Extrakt wurde nach einer Modifikation des Verfahrens von Krupey et al. (Immunochem. 9, 617, 1972) gewonnen:

Gepooltes Tumorgewebe (Primärtumoren: Colonkarzinom u.a.; Lebermetastasen; pro Extraktion ca. 500 - 1000 g Feuchtgewicht) wird bei -70°C aufbewahrt, zur Verarbeitung aufgetaut und mit Schere und Messer grob vorzerkleinert, anschließend mit einem maschinellen Zerkleinerer (Braun-MULTIMIX, VIRTIS-Homogenizer "60" d. Fa. Cenco: 30.000 UpM, 1 h, 4°C) mit etwa dem gleichen Volumen sterilem dest. Wasser weiterverarbeitet und zuletzt mit einem Gerät nach Potter-Elvehjem (60 ml-Gefäß, Fa. Braun) und einem motorgetriebenen Glaspistill zu einem feindispersen Brei homogenisiert. Dieses Gewebebrei-Gemisch oder stark CEA-haltige Körperflüssigkeit (z. B. Pleura-Exsudat oder Ascites, CEA-Konz. über 1000 ng/ml) wird mit einem gleichen Volumen 1,2 m Perchlorsäure versetzt und über 30 Minuten bei Raumtemperatur (R.T.) gerührt. Nach Zentrifugation (1000 g, 20 - 30 Minuten, R.T.) wird das Präzipitat verworfen und der Überstand bei klarem Aussehen sofort oder bei trübem Aussehen (fetthaltig) erst nach Vorfiltration ("Blauband"-Filter Nr. 589, Fa. Schleicher & Schüll) in Dialyse-Schläuche (Fa. Kalle, Niederlassung der HOECHST AKTIENGESELLSCHAFT, Kaliber 38, Länge bis zu 100 cm) überführt und in diesen in einem Behälter mit fließendem Leitungswasser und ständigem Zu- und Ablauf (1 - 3 l/Minute) für 24 - 48 Stunden dialysiert. Danach wird das Diaysat (ca. 1 - 3 l) mit $NaN_3$ (0,02 %) versetzt und mittels einer Konzentrier-Vorrichtung (Fa. Amicon, Ultrafiltrationszelle UF 400 mit Dialysiermembran PM 10, Reservoir von 4 l) auf ca. 100 ml eingeengt, nochmals auf das 10-fache Volumen mit sterilem bidestilliertem Wasser aufgefüllt und wieder auf 100 ml eingeengt. Dieses Endkonzentrat wird zentrifugiert (10 000 g, 1 h, $4^{\circ}C$) und der Überstand nach Ultrafiltration (SARTORIUS-Filter Ø 25 mm, 0,2, 0,6, und 3,0 /um) lyophilisiert. Der aus einer Ausgangsmenge von 500 - 1000 g Feuchtgewebe erhaltene Perchlorsäure-Extrakt ergibt ein Endgewicht von 500 bis 5000 mg.

## Beispiele

1. Gelfiltration

250 - 350 mg Extrakt werden über eine Säule (2,5 · 80 cm) gelfiltriert, die mit mit N,N'-Methylen-bisacrylamid quervernetztem Allyl-dextran mit einem Perlendurchmesser von 40 - 105 /um und einem Proteinfraktionierbereich von $2 \cdot 10^4$ bis $8 \cdot 10^6$ ($^{(R)}$SEPHACRYL S 400, Fa. Pharmacia) beschickt ist (Säule 1), unter Verwendung von 0,05 m Tris-HCl-Puffer vom pH 6,5 (Tris-(hydroxymethyl)-aminomethan-Hydrochlorid; Puffer 1). Die Elutionsgeschwindigkeit betrug 20 - 25 ml/h; gesammelt wurden Einzelfraktionen zu 5 ml. Es ergab sich folgendes Elutionsdiagramm (Messung der Transmission bei 280 nm):

1. Kleiner Vorpeak (Fraktion 1),

2. Zwischenzone (Fraktion 2),

3. breiter Hauptpeak mit steilem Anstieg (Fraktion 3) und

4. flacherem Abfall (Fraktion 4).

Die aufgefangenen Einzelfraktionen zu 5 ml wurden im Immunodiffusionstest mit Anti-CEA-Referenz-Antiserum bzw. im CEA-RIA getestet und zu den vorstehend genannten Sammelfraktionen vereinigt.

Fraktion 1 ist frei von CEA (und NCA), in den Fraktionen 2 bis 4 nimmt der Anteil von CEA sukzessive ab und der von NCA ab Fraktion 3 zu.

Wenn erwünscht, kann durch Rechromatographie der Fraktionen 2 bis 4 (einzeln oder vereinigt) eine bessere Trennung von CEA- und NCA-haltigen Einzelfraktionen erreicht werden. Hierzu dient eine SEPHACRYL S 200-Säule (2,5 $\cdot$ 80 cm, Proteinfraktionierbereich von 5 $\cdot$ $10^3$ bis 2,5 $\cdot$ $10^5$; Säule 2). Hierbei erhält man die Fraktionen 5 - 7, wobei Fraktion 5 hauptsächlich CEA und die Fraktionen 6 und 7 hauptsächlich NCA enthalten.

Die Fraktionen 2 bis 4 bzw. 5 bis 7 werden in einer Konzentrier/Dialysier-Vorrichtung (Einengungsgefäß mit Kollodiumhülsen, Fa. Sartorius) gegen einen Puffer (0,15 m Natriumchlorid in 0,02 m Natriumphosphatpuffer pH 7,3 mit 0,02 % Natriumazid; Puffer 2) dialysiert und auf 0,5 bis 1,5 ml eingeengt.

Verwendet man anstelle der Säule 1 eine Agarosesäule $^{(R)}$SEPHAROSE 6 B (Fa. Pharmacia; Säule 3), so erhält man ebenfalls gute Ergebnisse.

Setzt man anstelle der Säule 2 eine $^{(R)}$ SEPHADEX G 200- Säule (Fa. Pharmacia; Säule 4) ein, so wird eine vergleichbare Trennung erzielt.

## 2. Affinitäts-Chromatographie (Immunadsorption)

Das für die Immunadsorptionssäule benötige Anti-CEA-Antiserum wurde durch Immunisierung von Kaninchen mit hochgereinigtem CEA bzw. vorgereinigtem CEA-haltigem Perchlorsäure-Extrakt hergestellt. Es wurde mit lyophilisiertem Normalserum (etwa 10 mg/ml) und gewaschenen humanen A/B-Erythrozyten (nach Dekomplementierung des Antiserums) bis zur Nicht-Reaktivität (Immunodiffusion, mikroskopischer Test) absorbiert.

Nach abgeschlossener Absorption des Antiserums erfolgt eine IgG-Präparation durch kontinuierliche Anionenaustauscher-Chromatographie an einer DEAE-SEPHACEL[(R)]-Säule (Diethylaminoethyl-Cellulose, Fa. Pharmacia; Säule 5). Als Startpuffer wird 0,01 m Natriumphosphatpuffer pH 8,0 (Puffer 3) und als Grenzpuffer 0,5-m Natriumphosphatpuffer pH 4,5 (Puffer 4) benutzt, wobei mit einem extrem konkaven pH- und Molaritätsgradienten (z.B. mittels ULTROGRAD Gradientenmischer, Fa. LKB) eluiert wird. Die Auswahl der CEA-reaktiven reinen Anti-CEA-IgG-Fraktionen (Anfangsfraktionen; Fraktion 8) erfolgt nach Immunodiffusions- bzw. Immunelektrophorese-Test, zeitlich später eluierte CEA-reaktive Antikörper, die jedoch mit anderen Proteinen verunreinigt sind, werden getrennt gesammelt und als Antiseren für die Immunodiffusion bzw. Immunelektrophorese verwendet (Fraktion 9).

Zur Herstellung der Immunadsorptionssäule wird eine Säule (1,5 · 20 cm) mit 10 - 20 g eines vernetzten Dextrans (Bromcyan-aktivierte SEPHAROSE 4 B, Fa. Pharmacia; Säule 6) und angekoppelten 100 - 200 mg des chromatographisch gereinigten IgG-Globulins des Anti-CEA-Antiserums (Fraktion 8) beschickt. Auf diese Säule werden 2 - 4 ml der Fraktionen 2 - 4 bzw. 5 - 7 gegeben und langsam (20 sec/Tropfen) mit Puffer 2 im Lauf von 2 - 4 Stunden eluiert (Transmissionsmessung bei 280 nm). Nach vollständiger
Elution der nicht-gebundenen Proteine werden die Antikörper-
gebundenen Proteine mit einem Desorptionspuffer (Puffer 2
mit 2,0 m KSCN, pH 7,3; Puffer 5) eluiert (10 sec/Tropfen).
Dabei erscheint im Elutionsdiagramm ein hoher spitzer
Gradient, der bei weiterer Elution allmählich auf ein
Plateau abfällt, das wegen der KSCN-Transmissionsbeeinflussung bei 280 nm deutlich über dem Ausgangsniveau (100 %
Transmissionslinie) liegt. Nach vollständiger Elution
(Plateaulinie parallel zur 100 %-Linie) wird die Säule mit
Puffer 2 regeneriert, wobei im Elutionsdiagramm die Plateaulinie wieder auf die 100 %-Ausgangslinie der Transmissionsmessung abfällt.

Aufgefangen werden die folgenden Fraktionen:

Fraktion 10 (nicht gebundene Proteine)
Fraktion 11 (Zwischenfraktion) und
Fraktion 12 (gebundene Proteine). Fraktion 12 enthält intaktes CEA, CEA-Bruchstücke und NCA.

Die Fraktion 10 wird auf 0,5 - 1,0 ml durch Dialyse gegen
Puffer 2 (Kollodiumhülsen) konzentriert und auf CEA/NCA
getestet. Fraktion 11 wird verworfen. Fraktion 12 wird gegen
0,05 m Natriumphosphatpuffer pH 7,5 (Puffer 6) dialysiert
und auf 50 - 200 $\mu$l eingeengt.

Wenn die Säule 6 mit Anti-CEA-IgG-Antikörpern eines
Antiserums beladen wird, das zuvor mit normalem Milz-
bzw. Lungengewebsextrakt (stark NCA-haltig) absorbiert
wurde und deshalb frei von Antikörpern gegen NCA ist,
enthält Fraktion 12 in diesem Fall kein NCA mehr.

Wenn Fraktion 12 wegen unspezifischer Adsorption aus Säule
6 noch Serumproteinverunreinigungen (z.B. Albumin, $alpha_1$-
Antitrypsin, saures $alpha_1$-Glykoprotein) aufweist, können

diese durch Immunadsorption über eine weitere Säule 7 mit angekoppelten IgG-Antikörpern gegen humane Serumproteine bzw. perchlorsäureextrahierte Serumproteine entfernt werden, wobei dann die zuerst eluierte Fraktion (Fraktion 13) serumproteinfreies CEA/NCA enthält.

Die zur Weiterverarbeitung verwendeten Fraktionen enthalten neben intaktem CEA und NCA noch CEA-Bruchstücke.

3. Hochleistungschromatographie

Die abschließende Isolierung von CEA, NCA und CEA-Bruchstücken erfolgt durch hochauflösende sterische Ausschluß-Chromatographie mit einer HPLC-Säule Ultro-Pac TSK G 4000 SW, 7,5 ' 600 mm, Proteinfraktionierbereich 5000 - 1 000 000, Fa. LKB; Säule 8). Verwendet wurde eine HPLC-Vorrichtung der Fa. LKB (Multi-PERPEX-Pumpe, UV-Durchflußfotometer UVICORD S, Fraktionssammler Ultro-RAC II, 2-Kanal-Flachbettschreiber). Aufgetragen werden 50 - 500 /ug Protein in 50 - 250 /ul der eingeengten Fraktion 12. Eluiert wird mit Puffer 6 bei einer Elutionsgeschwindigkeit von 6 ml/Stunde in 350 /ul-Fraktionen über 5 Stunden Gesamttrennzeit.

Im Elutionsdiagramm (Messung von Transmission und Absorption mit variabler Empfindlichkeit von 0,01 bis 0,1 bei 280 nm) finden sich 3-Haupt-Peaks, wobei der mittlere Peak eine Schulter aufweist, d.h. aus mindestens 2 Unterfraktionen besteht.

Entsprechend dem Elutionsdiagramm wurden die folgenden Fraktionen gesammelt:

Fraktion 14 (Hauptfraktion, CEA-reaktiv),
Fraktion 15 (CEA-reaktiv) und
Fraktion 16 (NCA-reaktiv und CEA-reaktiv).

Nach einer Kalibrierung der HPLC-Säule mit Referenz-Proteinen (Thyroglobulin (MG 330 000) bis zu alpha-Lactalbumin MG 14 400)) können die Molekulargewichte der erhaltenen Fraktionen wie folgt abgeschätzt werden:

Fraktion 14: 250 000 - 150 000 D
Fraktion 15: 150 000 -  80 000 D
Fraktion 16:  80 000 -  40 000 D.

Nach Konzentration auf 100 bis 200 /ul in Puffer 6 können die Fraktionen unmittelbar für eine Radiojodmarkierung nach dem Chloramin T-Verfahren verwendet werden. Sie können auch auf einer SEPHADEX G 25-Säule (2,0 · 20 cm; Säule 9) entsalzt und anschließend lyophilisiert werden, wobei Endproteinmengen zwischen 100 und 1000 /ug erhalten werden.

Fraktion 14 enthält hochgereinigtes intaktes CEA und Fraktion 16 intaktes NCA, während Fraktion 15 immunoreaktive CEA-Bruchstücke enthält, die z. B. noch zur Immunisierung verwendet werden können.

## 4. Jodierung von CEA

20 /ug CEA in 20 /ul 0,05 M Natriumphosphatpuffer vom pH 7,4 (im folgenden "Puffer 7") werden mit etwa 1,5 mCi Na-$^{125}$J (abgepuffert mit 10 /ul 0,5 M Puffer 7) vermischt und nach Zugabe der Chloramin T-Lösung (20 /ug Chloramin T in 100 /ul 0,05 M Puffer 7) wird das Reaktionsgemisch 60 Sekunden gerührt. Danach wird die Reaktion durch Zusatz von Natriummetabisulfit (20 /ug $Na_2S_2O_5$ in 50 /ul 0,05 M Puffer 7) gestoppt. Es wird weitere 30 Sekunden gemischt und zur Stabilisierung 0,2 ml Rinderserumalbumin-Lösung (1 % Rinderserumalbumin, 0,05 M Puffer 7, 0,1 % $NaN_3$) zugegeben.

Die Reinigung des $^{125}$J-markierten CEA erfolgt durch Hochleistungschromatographie-Trennung des Jodierungsgemisches über eine Säule 8.

Elutionspuffer: 0,05 M Puffer 7, 0,1 % $NaN_3$; Fluß: 0,1 ml/min.

Spez. Aktivität: ca. 30 mCi/mg, Immunreaktivität: ca. 90 %

Patentansprüche:

1. Carcinoembryonales Antigen (CEA), erhältlich aus CEA enthaltenden Perchlorsäureextrakten durch

   a) Abtrennung höhermolekularer Proteine durch Gelfiltration,
   b) Selektionierung der Proteine mit CEA-Antigenität aus dem Eluat durch Immunadsorption und
   c) Auftrennung durch Flüssigchromatographie mit hochauflösenden Säulen.

2. Verfahren zur Isolierung von CEA aus CEA enthaltenden Perchlorsäureextrakten, dadurch gekennzeichnet, daß

   a) höhermolekulare Proteine durch Gelfiltration abgetrennt werden,
   b) aus dem Eluat durch Immunadsorption Proteine mit CEA-Antigenität selektioniert werden, die
   c) durch Flüssigchromatographie mit hochauflösenden Säulen aufgetrennt werden.

3. Verfahren nach Anspruch 2, dadurch gekennzeichnet, daß die höhermolekularen Proteine durch Gelfiltration an quervernetzten Dextranen abgetrennt werden.

4. Verfahren nach Anspruch 2 oder 3, dadurch gekennzeichnet, daß zur Immunadsorption eine Säule mit IgG-Globulinen eines Anti-CEA-Antiserums beladen wird.

5. Verfahren nach einem oder mehreren der Ansprüche 2 bis 4, dadurch gekennzeichnet, daß durch Flüssigchromatographie mit hochauflösenden Säulen CEA als Fraktion mit einem Molgewichtsbereich von 250 000 bis 150 000 abgetrennt wird.

6. Verfahren nach einem oder mehreren der Ansprüche 2 bis 5, dadurch gekennzeichnet, daß das CEA enthaltende Eluat der Flüssigchromatographie nach Konzentration für eine Radiojodmarkierung eingesetzt wird.

7. Verfahren nach Anspruch 6, dadurch gekennzeichnet, daß die Radiojodmarkierung nach dem Chloramin T-Verfahren erfolgt.

8. Radiojodmarkiertes CEA, erhältlich nach Anspruch 6 oder 7.

9. Verwendung des CEA nach Anspruch 1 zur Herstellung von Diagnostika.

10. Diagnostikum, enthaltend ein radiojodmarkiertes CEA nach Anspruch 8.

- 1 - 0102008

<u>Patentansprüche für Österreich:</u>

1. Verfahren zur Isolierung von carcinoembryonalem Antigen (CEA) aus CEA enthaltenden Perchlorsäureextrakten, dadurch gekennzeichnet, daß

   a) höhermolekulare Proteine durch Gelfiltration abgetrennt werden,
   b) aus dem Eluat durch Immunadsorption Proteine mit CEA-Antigenität selektioniert werden, die
   c) durch Flüssigchromatographie mit hochauflösenden Säulen aufgetrennt werden.

2. Verfahren nach Anspruch 1, dadurch gekennzeichnet, daß die höhermolekularen Proteine durch Gelfiltration an quervernetzten Dextranen abgetrennt werden.

3. Verfahren nach Anspruch 1 oder 2, dadurch gekennzeichnet, daß zur Immunadsorption eine Säule mit IgG-Globulinen eines Anti-CEA-Antiserums beladen wird.

4. Verfahren nach einem oder mehreren der Ansprüche 1 bis 3, dadurch gekennzeichnet, daß durch Flüssigchromatographie mit hochauflösenden Säulen CEA als Fraktion mit einem Molgewichtsbereich von 250 000 bis 150 000 abgetrennt wird.

5. Verfahren nach einem oder mehreren der Ansprüche 1 bis 4, dadurch gekennzeichnet, daß das CEA enthaltende Eluat der Flüssigchromatographie nach Konzentration für eine Radiojodmarkierung eingesetzt wird.

6. Verfahren nach Anspruch 5, dadurch gekennzeichnet, daß die Radiojodmarkierung nach dem Chloramin T-Verfahren erfolgt.